# EUROPÄISCHE PATENTANMELDUNG

(11) **EP 0 916 361 A1**
(43) Veröffentlichungstag der Anmeldung: **19.05.1999**
(21) Anmeldenummer: 98120958.8
(22) Anmeldetag: 05.11.1998
(51) Int. Cl.: A61M 25/02

(54) **Armband**

(30) Priorität: 17.11.1997 DE 19750581
(71) Anmelder: Krütten, Viktor, D-65510 Idstein (DE)
(72) Erfinder: Krütten, Viktor, D-65510 Idstein (DE)
(74) Vertreter: Müller, Eckhard, Dr.

(57) **Zusammenfassung**

Die Erfindung betrifft ein Armband (2) mit Halterungen (4) für einen Verlängerungsschlauch (10), der mit zwei Verbindungsstücken (Lueranschlüssen 12, 14) versehen ist. Dieser Schlauch (2) wird zwischen ein Infusionsgerät und einen Katheter gelegt und erlaubt dem Patienten ein Öffnen, Schließen oder Wechseln der Infusion ohne fremde Hilfe

## Beschreibung

Die Erfindung betrifft ein Armband für medizinische Zwecke mit einem Verlängerungsschlauch, welcher zwischen eine intravenöse Verweilkanüle oder einen Katheter und ein Infusionsgerät geschaltet wird.

Bei Patienten, die parenteral ernährt oder medikamentös über Katheter oder intravenöse Verweilkanülen behandelt werden, wird üblicherweise der Katheter an der Hand oder in der Ellbogenbeuge angelegt, wobei dann an diesem, stets am Patienten verbleibenden Katheter der Schlauch des Infusionsgerätes angeschlossen wird. Der Katheter ist mittels Klebeband auf der Haut fixiert. Dabei ist zu vermeiden, daß sich der Katheter verschiebt, da solche Bewegungen zu Venenreizungen oder zu Thrombosen führen können. Im Extremfall kann der Katheter ganz herausgezogen und muß dann neu gesetzt werden. Dabei besteht die Gefahr des Entblutens oder einer Luftembolie. Es ist also eine Zugbeanspruchung auf die intravenöse Verweilkanüle oder den Katheter auf alle Fälle zu vermeiden.

Damit sich die Verbindung zwischen dem Schlauch des Infusionsgeräts und dem Katheter nicht versehentlich löst, ist die früher gebräuchliche Steckverbindung durch sogenannte Luer-Anschlüsse oder Luer-Lock-Verbindungen ersetzt worden. Bei diesen Verbindungen wird eine Art Stecker mit Außengewinde in eine mit Innengewinde versehene Buchse geschoben und durch Drehung festgeschraubt. Dabei schraubt sich das am Stecker vorhandene Gewinde in das Gegengewinde der Buchse und führt so zu einem sicheren, dichten Sitz, der ein versehentliches Öffnen der Verbindung - mit den Gefahren Luftembolie oder Entbluten - verhindert.

Beim derzeitigen Stand der Technik ist die Verbindung zwischen intravenöser Verweilkanüle und dem Infusionsschlauch direkt am Ende der intravenösen Verweilkanüle vorgesehen. Diese Stelle ist für den Patienten schlecht zugänglich, da die Kanüle direkt an der Haut befestigt ist. Insbesondere kann er nur mit einer Hand, nämlich der freien Hand, dorthin langen. Ein Öffnen des Verschlusses durch Verdrehen der Art Überwurfmutter der Buchse bei gleichzeitigem Festhalten des Gegenstückes und Herausziehen des Verlängerungsschlauches ist dadurch äußerst umständlich und Kann zu Fehlbedienungen führen. Beim heutigen Stand der Technik kann ein Patient also praktisch kaum das Verlängerungsstück ohne fremde Hilfe anschließen oder abziehen.

Aufgabe der Erfindung ist es daher, eine Vorrichtung vorzuschlagen, mit Hilfe derer der Patient selbsttätig die Verschlüsse zwischen Infusionsgerät und intravenöser Verveilkanüle oder Katheter bedienen kann, ohne daß die Gefahr eines Herausziehens des Katheters aus der Vene besteht.

Diese Aufgabe wird erfindungsgemäß gelöst mit einem Armband, das eine Halterung für einen Verlängerungsschlauch aufweist, wobei der Verlängerungsschlauch selbst zwei Verbindungsstücke aufweist. Diese Verbindungsstücke sind insbesondere Lueranschlüsse (Stecker und Buchse), so daß die Vorteile des Luersystems bezüglich der sicheren Verbindung beizubehalten sind. Ausgestaltungen der Erfindung sind Gegenstände von Unteransprüchen.

Mit der Erfindung ist es dem Patienten nunmehr möglich, den Verlängerungsschlauch vom Armband abzunehmen und damit die zu lösende Verbindung an eine Stelle zu bringen, an der er mit beiden Händen zufassen kann. Der Patient kann dann die Verbindung öffnen und einen neuen Infusionsschlauch anschließen oder den Infusionsschlauch wechseln. Da der Patient nun mit beiden Händen frei zugreifen kann, ist es für ihn leicht, den Lueranschluß auf- und zuzuschrauben, ohne daß irgendwelche Zugbelastungen auf die Schlauchverbindungen oder den Katheter wirken.

Ein wesentlicher Vorteil der Erfindung ist auch, daß der Patient eigenhändig den Anschluß schließen und öffnen kann, ohne daß es zu einer Zugbeanspruchung des Katheters kommt. Ein Abkoppeln von der Infusion oder ein Wechseln ist somit ohne fremde Hilfe möglich. Vorteilhaft ist, daß der in das Armband eingelegte Verlängerungsschlauch nicht hervorsteht. Damit kann sich der Patient z.B. leicht be- oder entkleiden, ohne behindert zu werden. Auf der anderen Seite wird vermieden, daß durch zufällige Zugbeanspruchungen der Schlauch aus seiner Verbindung oder aus der Kanüle gerissen wird.

Bevorzugt enthält das erfindungsgemäße Armband neben den Halterungen, wie Schnappelemente oder ähnlich wirkende, lösbare, formschlüssige Halter für den Schlauch, eine oder zwei ebenfalls lösbare Halterungen für einen oder für beide Verbindungsstücke (Lueranschlüsse) des Verlängerungsschlauches.

Günstig ist, wenn am Armband eine Halterung für den Lueranschluß vorgesehen ist, an dem die intravenöse Verveilkanüle oder der Katheter angeschlossen wird. Das Armband hält dann nämlich den Katheteranschluß starr in der gewünschten Position gegenüber der Vene, in die die Kanüle eingesteckt ist. Dadurch wird vermieden, daß Bewegungen in die Kanüle eingeleitet werden, die zu Venenreizungen oder Thrombosen führen könnten. Das fest am Körper angelegte erfindungsgemäße Armband hält so die Einstichstelle frei von Scher- und Zugbeanspruchungen.

Von Vorteil ist auch, wenn am Armband eine Halterung für das zweite Ende des Verlängerungsschlauches vorgesehen ist. Damit ist auch diese Verbindungsstelle geschützt an dem fest am Körper gelagerten Armband befestigt und liegt in einem geschützten Bereich nahe dem Körper.

Bevorzugt sind die Halterungen am Armband so ausgelegt, daß der Verlängerungsschlauch mäanderartig gehalten ist. Dies ist zum Beispiel dadurch realisiert, daß der Schlauch abwechselnd an den Seitenrändern des Armbandes gehaltert ist, so daß er platzssparend die gesamte Breite des Armbandes ausnutzend aufgenommen ist. Diese mäanderformige Aufbewahrung des Verlängerungsschlauches führt zu einem sicheren Verwahren direkt an der Haut, so daß er beim Aus- oder Umziehen nicht abgestreift wird.

Die Halterungen für den Verlängerungsschlauch können aus Kunststoff gefertigte Elemente sein, welches als Schnappelemente zum Halten des Verlängerungsschlauches oder der Lueranschlüsse ausgebildet ist. In diese Schnappelemente kann der Patient dann selbst den Schlauch einlegen oder für das Auswechseln herausnehmen. Dabei halten die Schnappelemente den Schlauch ausreichend fest am Armband, so daß ein unbeabsichtigtes Abstreifen verhindert ist.

Der Übergang zwischen dem mäanderförmig verlegten Verlängerungsschlauch und dem Lueranschluß, z.B. der Buchse, erfolgt über eine Abwinklung, die nicht nach außen vorsteht, sondern in Umfangsrichtung des Armbandes geführt ist. Dadurch wird vermieden, daß Teile vorstehen und evtl. bei unbedachten Bewegungen abgerissen werden können.

Das Armband kann aus einem beliebigen Material hergestellt sein, es sollte jedoch eine gewisse Flexibilität aufweisen, hautverträglich und luftdurchlässig sein, damit es zu keinen Hautreizungen durch Schweiß kommt. Zur Befestigung am Arm empfiehlt sich ein Klettverschluß, der ein Anpassen des Armbandes an unterschiedlich kräftige Arme leicht ermöglicht.

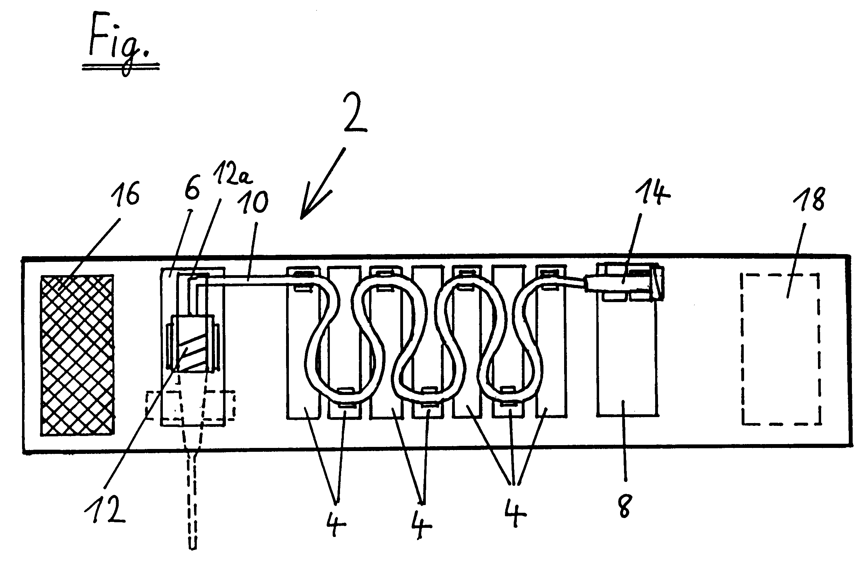

Weitere Ziele, Vorteile, Merkmale und Anwendungsmöglichkeiten der vorliegenden Erfindung ergeben sich aus der nachfolgenden Beschreibung eines Ausführungsbeispieles anhand der Zeichnung.

Dabei bilden alle beschriebenen und/oder bildlich dargestellten Merkmale für sich oder in beliebiger Kombination den Gegenstand der vorliegenden Erfindung auch unabhängig von ihrer Zusammenfassung in den Ansprüchen oder deren Rückbeziehung.

Die einzige Figur zeigt eine Ausführungsform eines erfindungsgemäßen Armbandes 2 in aufgeklappter Stellung mit den Halterungen 4 für den Verlängerungsschlauch 10 und der Halterung 6 für den als Buchse ausgebildeten Lueranschluß 12 und der Halterung 8 für den als Stecker ausgebildeten Lueranschluß 14. Der Verlängerungsschlauch 10 ist mäanderförmig zwischen den Schnappelementen der Halterungen 4 eingeklemmt. Dadurch kann der relativ lange Verlängerungsschlauch 10 auf dem begrenzten Platz des Armbands 2 untergebracht werden. An einem Ende des Verlängerungsschlauches 10 ist der Lueranschluß 12 bzw. die Buchse für die Verbindung mit der gestrichelt gezeigten intravenösen Verveilkanüle oder dem Katheter vorgesehen. Der Lueranschluß 12 weist eine Abwinklung 12a auf, die den Schlauch 10 faßt. Diese Verbindung ist besonders stabil und dauerhaft ausgelegt, was durch eine Klebung erreicht ist. Auf der gemäß der Figur linken Seite des Armbandes 2 ist der eine Teil 16 eines Klettverschlusses vorgesehen, die mit einem Gegenstück 18 zusammenwirkt, wenn das Armband 2 um den Arm gelegt ist. Das Gegenstück 18 befindet sich auf der rechten Seite des Armbandes 2, jedoch auf der Unterseite, und ist daher nur gestrichelt gezeichnet.

Die Funktion des erfindungsgemäßen Armbandes 2 gestaltet sich wie folgt:
Das Armband 2 wird dem Patienten zunächst so angelegt, daß die Halterung 6 für den Lueranschluß 12 bzw. die Buchse sich in etwa in dem Bereich befindet, in dem der Katheter oder die intravenöse Verweilkanüle eingesetzt werden soll. Dann setzt der Arzt die Kanüle oder den Katheter und verschraubt die Überwurfmutter des Lueranschlusses 12 mit dem Katheter. Zur Fixierung des Katheters sind nun keine Klebestreifen am Körper mehr nötig, sondern es kann im Schnappverschluß der Halterung 6 eingerastet werden. Die Einstichstelle ist dann frei von Zugund Schubbelastungen, da solche Belastungen durch das Armband 2 aufgenommen werden. An das andere Ende des Schlauches 10, nämlich den Lueranschluß 14 bzw. den Stecker schraubt der Arzt dann den normalen Schlauchanschluß bzw. der Buchse eines handelsüblichen Infusionsgerätes, welches normalerweise auch einen Bakterienfilter, einen Partikelfilter, eine Tropfkammer und eine Reguliermechanismus für die Regelung des Durchflusses aufweist. Auch dieser Anschluß ist durch die erfindungsgemäße Halterung 8 sehr gut geschützt, da er direkt am Armband befestigt ist und damit dem Wirkungsbereich des Patienten entzogen, wo durch unbedachte Handlungen ein unbeabsichtigtes Lösen erfolgen könnte.

Will der Patient nun die Verbindung am Lueranschluß lösen, weil er z.B. ein anderes Infusionsgerät anschließen will, kann er den Anschluß 14 mit der freien Hand aus dem Schnappverschluß nehmen, anheben, den Verlängerungsschlauch 10 aus den ihn haltenden Schnappelementen der Halterungen 4 heraus nehmen und soweit ziehen, daß der Lueranschluß 14 in die Nähe der Finger der Hand gebracht wird, an der das Armband 2 befestigt ist. Mit beiden Händen kann der Patient dann den Schraubverschluß am Lueranschluß 14 lösen und ein anderes Infusionsgerät anschließen. Nach dem Festschrauben, was mittels beider freier Hände besonders einfach und sicher gelingt, wird Anschluß 14 wieder am Armband 2 befestigt und der Verlängerungsschlauch 10 mit den Fingern der anderen Hand wieder mäanderförmig in die Schnappelemente der Halterung 4 eingebracht.

## Patentansprüche

1. Armband (2), gekennzeichnet durch Halterungen (4) für einen Verlängerungsschlauch (10), der mit zwei Verbindungsstücken (Lueranschlüssen 12, 14) versehen ist.

2. Armband (2) nach Anspruch 1, gekennzeichnet durch eine oder zwei Halterungen (6, 8) für den oder die Verbindungsstücke (Lueranschlüsse) (12, 14).

3. Armband (2) nach Anspruch 1 oder Anspruch 2, dadurch gekennzeichnet, daß die einen Halterungen (4) den Verbindungsschlauch (10) meanderartig halten.

4. Armband (2) nach einem der vorhergehenden Ansprüche, dadurch gekennzeichnet, daß die Halterungen (4) abwechselnd im Bereich der Seitenränder des Armbandes (2) angeordnet sind.

5. Armband (2) nach einem der vorhergehenden Ansprüche, dadurch gekennzeichnet, daß die Halterungen (4, 6, 8) aus Kunststoff gefertigte Elemente sind, die Schnappelemente zum Halten des Verlängerungsschlauches (10) oder der Lueranschlüsse (12, 14) aufweisen.

6. Armband (2) nach einem der vorhergehenden Ansprüche, gekennzeichnet durch einen Klettverschluß (16, 18) zum Schließen des Armbandes (2).
